# EUROPEAN PATENT APPLICATION

(11) **EP 4 470 503 A1**
(43) Date of publication of application: **04.12.2024**
(21) Application number: 22937251.1
(22) Date of filing: 01.12.2022
(51) Int. Cl.: A61F 2/24

(54) **ARTIFICIAL HEART VALVE**

(30) Priority: 14.04.2022 CN 202210396030
(71) Applicant: Shanghai Trulive Medtech Co., Ltd., Shanghai 201206 (CN); Jiangsu Trulive Medtech Co., Ltd., Nantong, Jiangsu 226400 (CN)
(72) Inventor: HAN, Tianyu, Shanghai 201206 (CN); LIU, Xiang, Shanghai 201206 (CN); WEI, Yongqiang, Shanghai 201206 (CN)
(74) Representative: Nordmeyer, Philipp Werner
(86) International application number: PCT/CN2022/135853
(87) International publication number: WO 2023/197619

(57) **Abstract**

The present invention provides an artificial heart valve comprising a main stent and an artificial valve leaflet, wherein an outflow end of the main stent is connected to an inflow end of the artificial valve leaflet, and when the artificial heart valve is implanted in the heart, the main stent is fixed on a mitral valve annulus above a native posterior leaflet of mitral valve leaflets, and the artificial valve leaflet is sealed against an inner side of the native posterior leaflet and mates with a native anterior leaflet to ensure a unidirectional flow of blood in the left atrium and the left ventricle; wherein, the artificial valve leaflet comprises a valve leaflet stent and a covering, and wherein the valve leaflet stent is a single-layer three-dimensional stent, and the covering covers the valve leaflet stent. The present invention utilizes the artificial valve leaflet for occupation and mating and effectively utilizes the healthy native anterior leaflet, thereby improving the valve leaflet's mating performance and lifespan. The artificial heart valve of the present invention has a compact structure and a small volume, and after implantation, it only occupies the area where the posterior leaflet is, which has little effect on the heart's function, and occupies a small area of the valve orifice, so the impact of blood flow is small, thereby improving the lifespan of the artificial heart valve.

## Description

### TECHNICAL FIELD

The present invention relates to the technical field of medical devices, and in particular to an artificial heart valve.

### BACKGROUND

The heart has four chambers, the left atrium (LA) and left ventricle (LV) on the left side of the heart, and the right atrium (RA) and right ventricle (RV) on the right side of the heart. The ventricular inflow tract is formed between the atrium and ventricle, the left ventricular outflow tract is formed between the left ventricle and the aorta, and the right ventricular outflow tract is formed between the right ventricle and the pulmonary artery. During the entire cardiac cycle, the pumping action of the left atrioventricular and right atrioventricular of the heart generally occurs synchronously. At the same time, everyone has four valves in their heart, namely the aortic valve connecting the left ventricle and aorta, the pulmonary valve connecting the right ventricle and pulmonary artery, the mitral valve connecting the left atrium and left ventricle, and the tricuspid valve connecting the right atrium and right ventricle. They all act as one-way valves, allowing blood to flow from one direction to another but not backwards. At the onset of ventricular filling (i.e., diastole), the aortic and pulmonary valves close to prevent backflow from the arteries into the ventricles; shortly thereafter, the mitral and tricuspid valves open to allow unimpeded flow from the atria into the respective ventricles. Shortly after the onset of ventricular systole (i.e., emptying of the ventricles), the tricuspid and mitral valves normally close to form a seal that prevents backflow from the ventricles into the corresponding atria.

The structure of the human heart is very complex, especially the mitral valve structure is more complex than the aortic valve. The shape of the mitral valve annulus is irregular, and the multiple chordae tendineae in the ventricular cavity seriously interfere with the implantation and positioning of the interventional valve. Therefore, transcatheter mitral valve replacement (TMVR) is a method of catheter intervention that compresses the artificial valve into a delivery system outside the body, and delivers it to the human mitral valve annulus along the vascular path or apex, and then releases and fixes the artificial valve at the mitral valve annulus to replace the native valve. Compared with surgical operations, TMVR does not require an extracorporeal circulation assist device. It has the advantages of less trauma and faster patient recovery. The patient's hemodynamic indicators can be significantly improved after surgery. Compared with the apical route, the transfemoral atrial septal approach is less traumatic and has a wider audience.

Although mitral valve replacement technology has developed rapidly, there are still some recognized difficulties in the design of artificial valves. For example, due to the complex structure of the mitral valve (saddle shape, large size, etc.), it is difficult to anchor the stent of the artificial valve; the existing stent for mitral valve replacement is structured to have a large size, which has a certain impact on the normal work of the heart; the performance problems of the artificial valve leaflet of the artificial valve (such as biological leaflets or mechanical leaflets) make the life of the existing artificial valve leaflet limited, especially the biological leaflets used in interventional treatment, such as porcine pericardium. When opened and closed for a long time, the mechanical properties of the artificial valve leaflet will be reduced or even fail due to factors such as impact load and friction, which not only makes its life shorter than that of the native leaflets, but also in the long run, the mating performance of the artificial valve leaflet is not as good as that of the native leaflets.

### SUMMARY OF THE INVENTION

An object of the present invention is to provide an artificial heart valve which can solve the problems of the existing artificial valve leaflet which is structured to have a large size, has a limited lifespan, and has a reduced mating performance of the artificial valve leaflet.

In order to solve the above problems, the present invention provides an artificial heart valve, comprising a main stent and an artificial valve leaflet, wherein an outflow end of the main stent is connected to an inflow end of the artificial valve leaflet, and when the artificial heart valve is implanted in the heart, the main stent is fixed on a mitral valve annulus above a native posterior leaflet of mitral valve leaflets, and the artificial valve leaflet is sealed against an inner side of the native posterior leaflet and mates with a native anterior leaflet of the mitral valve leaflets to ensure a unidirectional flow of blood in the left atrium and the left ventricle;
wherein, the artificial valve leaflet comprises a valve leaflet stent and a covering, and wherein the valve leaflet stent is a single-layer three-dimensional stent, and the covering covers the valve leaflet stent.

Optionally, the main stent has an arc-shaped structure; when the artificial heart valve is implanted in the heart, a surface of the artificial valve leaflet facing the native anterior leaflet matches in shape with a surface of the native posterior leaflet facing the native anterior leaflet.

Further, the main stent comprises a first section and a second section that are connected along a longitudinal direction, an inflow end of the first section is configured to be connected to a delivery system before the artificial heart valve is released, an outflow end of the first section is connected to an inflow end of the second section, and an outflow end of the second section is connected to an inflow end of the valve leaflet stent.

Further, the first section comprises at least one first fixing lug and two first ribs connected to each of the at least one first fixing lug, inflow ends of the two first ribs are connected together to one of the at least one first fixing lug, outflow ends of the two first ribs are respectively connected to the second section, the first fixing lug is configured to connect to the delivery system, and the outflow ends of the first ribs are connected to the inflow end of the second section.

Further, each of the first ribs extends curvedly from the inflow end to the outflow end of the first rib and bulges away from the inner side of the native posterior leaflet.

Further, the second section comprises a plurality of second ribs, and wherein after all the second ribs are connected end to end in sequence, inflow ends of the second ribs are connected to the outflow ends of the first ribs, and outflow ends of the second ribs are connected to the inflow end of the valve leaflet stent, so that the two first ribs connected to a same one of the at least one first fixing lug and corresponding two second ribs together form a closed mesh cell.

Further, it comprising at least one fixing stent, wherein each of the at least one fixing stent has an outflow end inserted into one of the at least one first fixing lug, and an inflow end connected to the delivery system;
after the artificial heart valve is implanted in the heart, the outflow end of the fixing stent is anchored on the mitral valve annulus above the native posterior leaflet, and the inflow end of the first section is fitted and fixed on the mitral valve annulus.

Further, the fixing stent comprises a connecting section and an anchoring section that are arranged in a longitudinal direction, wherein the connecting section is detachably connected to an inflow end of the anchoring section, and the connecting section is connected to the delivery system.

Further, the anchoring section comprises a peg and a pressing sheet, wherein the pressing sheet and the at least one first fixing lug are sleeved over the peg, the pressing sheet is arranged close to an inflow end of the peg, and the at least one first fixing lug is arranged close to an outflow end of the peg;
after the artificial heart valve is implanted in the heart, the outflow end of the peg pierces a native tissue of the mitral valve annulus, the at least one first fixing lug is longitudinally limited on the peg between the native tissue and the pressing sheet, and an inflow end of the main stent is arranged in contact with the native tissue.

Further, the outflow end of the peg has a sharp end, the peg has a largest cross-sectional diameter at an inflow end of the sharp end, and a smallest cross-sectional diameter at an outflow end of the sharp end.

Further, a serrated section is provided on a middle area of an outer circumferential surface of the peg, and the serrated section has a serrated structure; the pressing sheet is annular, and a side wall of an inner ring has a serrated structure matching the serrated structure of the serrated section, so that the pressing sheet can only move unidirectionally from an inflow end to an outflow end of the serrated section.

Further, the connecting section comprises a second fixing lug, a sleeve and a pin, wherein the second fixing lug is fixed to an end face of an inflow end of the peg and is configured to connect to a fixing pull wire of the delivery system, the sleeve is sleeved over an inflow end of the peg and is connected to the peg through the pin, and an end of the pin is connected to the unlocking pull wire of the delivery system.

Further, a first through hole extending through the peg laterally is defined on a side wall of the inflow end of the peg, two second through holes extending through the sleeve laterally is defined on a side wall of an outflow end of the sleeve, the pin is inserted into the first through hole and the second through hole, and can be withdrawn from the first through hole and the second through hole through the unlocking pull wire.

Optionally, the main stent comprises a plurality of barbs, and the artificial valve leaflet further comprises a clamping component, wherein all of the barbs and the clamping component are arranged on a same side, and the barbs are arranged at an outflow end of a first section of the main stent and an inflow end of the second section of the main stent, the clamping component is arranged at an outflow end of the valve leaflet stent, and the covering exposes the clamping component,
when the artificial heart valve is implanted in the heart, the main stent is anchored on the mitral valve annulus with an assistance of the barbs, and the clamping component clamps and fixes the native posterior leaflet between the clamping component and the valve leaflet stent.

Optionally, the valve leaflet stent comprises at least two sub-stents connected in sequence along a transverse direction, and two adjacent ones of the at least two sub-stents are symmetrically arranged with respect to a connection point.

Further, each of the at least two sub-stent comprises a third rib, a fourth rib and a fifth rib, wherein an inflow end of the third rib and an inflow end of the fourth rib are arranged at intervals and are both connected to an outflow end of a second section of the main stent, the fifth rib is located between the third rib and the fourth rib in the transverse direction, and an outflow end of the third rib and an outflow end of the fourth rib are respectively connected to two ends of the fifth rib.

Further, the third rib bulges away from the fourth rib, and/or the fourth rib bulges away from the third rib.

Further, the third rib is straight, and the fourth rib is curved, wherein the fourth rib extends away from the third rib in the transverse direction and away from the outflow end of the second section in a longitudinal direction, and then extends toward the third rib in the transverse direction and away from the outflow end of the second section in the longitudinal direction.

Optionally, the covering is filled with a filler.

Optionally, the clamping component is formed by bending a metal rod, and the clamping component has two ends, one end of which is connected to an outflow end of the valve leaflet stent, and the other end extends away from the valve leaflet stent in a transverse direction and away from an inflow end of the valve leaflet stent in a longitudinal direction, and then extends away from the valve leaflet stent in the transverse direction and toward the inflow end of the valve leaflet stent in the longitudinal direction.

Compared with the prior art, the present invention has the following beneficial effects:
The present invention provides an artificial heart valve comprising a main stent and an artificial valve leaflet, wherein an outflow end of the main stent is connected to an inflow end of the artificial valve leaflet, and when the artificial heart valve is implanted in the heart, the main stent is fixed on a mitral valve annulus above a native posterior leaflet of a mitral valve leaflets, and the artificial valve leaflet is sealed against an inner side of the native posterior leaflet and mates with a native anterior leaflet of the mitral valve leaflets to ensure a unidirectional flow of blood in the left atrium and the left ventricle; wherein, the artificial valve leaflet comprises a valve leaflet stent and a covering, and wherein the valve leaflet stent is a single-layer three-dimensional stent, and the covering covers the valve leaflet stent. The present invention utilizes the artificial valve leaflet for occupation and mating and effectively utilizes the healthy native anterior leaflet, thereby improving the valve leaflet's mating performance and lifespan. The artificial heart valve of the present invention has a compact structure and a small volume, and after implantation, it only occupies the area where the posterior leaflet is, which has little effect on the heart's function, and occupies a small area of the valve orifice, so the impact of blood flow is small, thereby improving the lifespan of the artificial heart valve.

In addition, the plurality of barbs, the clamping component and the anchoring section more reliably anchor the artificial heart valve between the left atrium and the left ventricle.

### BRIEF DESCRIPTION OF THE DRAWINGS

Fig. 1 is a schematic structural diagram of an artificial heart valve located in a heart according to an embodiment of the present invention;
Figs. 2a-2c are schematic diagrams of the three-dimensional structure of an artificial heart valve viewed from different perspectives according to an embodiment of the present invention;
Figs. 3a-3b are schematic diagrams of the three-dimensional structure of a stent body and a leaflet stent according to an embodiment of the present invention;
Figs. 4a-4c are schematic diagrams of an artificial heart valve during implantation according to an embodiment of the present invention;
Fig. 5 is a schematic diagram of a fixing stent according to an embodiment of the present invention.

### Description of reference numerals:

10-left atrium; 20-left ventricle; 21-native posterior leaflet; 22-native anterior leaflet; 30-delivery catheter; 31-fixing pull wire; 32-unlocking pull wire;
100-artificial heart valve; 110-main stent; 111-first section; 1111-first fixing lug; 1112-first rib; 112-second section; 1121-second rib; 120-artificial valve leaflet; 121-valve leaflet stent; 1211-third rib; 1212-fourth rib; 1213-fifth rib; 130-clamping component; 140-barb;
200-fixing stent; 210-connecting section; 211-second fixing lug; 212-sleeve; 213-pin; 220-anchoring section; 221-peg; 2211-sharp end; 2212-serrated section; 222-pressing sheet.

### DETAILED DESCRIPTION

The core of the present invention is to provide an artificial heart valve, including a main stent and an artificial valve leaflet, wherein the outflow end of the main stent is connected to the inflow end of the artificial valve leaflet, and when the artificial heart valve is implanted in the heart, the main stent is fixed on the mitral valve annulus above the native posterior leaflet of the mitral valve leaflets, and the artificial valve leaflet is sealed against the inner side of the native posterior leaflet and mates with the native anterior leaflet of the mitral valve leaflets to ensure the unidirectional flow of blood in the left atrium and the left ventricle;
wherein, the artificial valve leaflet includes a valve leaflet stent and a covering, wherein the valve leaflet stent is a single-layer three-dimensional stent, and the covering covers the valve leaflet stent.

The present invention utilizes artificial valve leaflet for occupation and mating and effectively utilizes the healthy native anterior leaflet, thereby improving the valve leaflet's mating performance and lifespan. The artificial heart valve of the present invention has a compact structure and a small volume, and after implantation, it only occupies the area where the posterior leaflet is, which has little effect on the heart's function, and occupies a small area of the valve orifice, so the impact of blood flow is small, thereby improving the lifespan of the artificial heart valve.

An artificial heart valve of the present invention will be described in further detail below. The present invention will be described in more detail below with reference to the accompanying drawings, in which preferred embodiments of the present invention are shown. It should be understood that those skilled in the art can modify the present invention described herein and still achieve the beneficial effects of the present invention. Therefore, the following description should be understood as being widely known to those skilled in the art, rather than as a limitation to the present invention.

In the interest of clarity, not all features of an actual implementation are described. In the following description, well-known functions or constructions are not described in detail since they would obscure the invention in unnecessary detail. It should be appreciated that in the development of any actual embodiment, numerous implementation details must be made to achieve the developer's specific goals, such as changing from one embodiment to another in accordance with constraints related to system or business. Moreover, it should be appreciated that such a development effort might be complex and time-consuming, but would nevertheless be a routine undertaking for those of ordinary skill in the art.

In order to make the purpose and features of the present invention more obvious and understandable, the specific implementation methods of the present invention are further described below in conjunction with the accompanying drawings. It should be noted that the drawings are all in very simplified form and use inaccurate ratios, and are only used to conveniently and clearly assist in illustrating the embodiments of the present invention. Herein, the term "or" is generally used in its sense including "and/or" unless the content clearly dictates otherwise. The terms "inner", "outer", and similar expressions used herein are for illustrative purposes only and do not represent the only implementations. Herein, the end close to where blood flows into the left atrium is defined as the inflow end, and the end close to where blood flows out of the left ventricle is defined as the outflow end.

The structure of the left heart includes the left atrium, the left ventricle, the mitral valve annulus between the left atrium and left ventricle, the mitral valve leaflets attached to the mitral valve annulus, and the papillary muscles and their chordae tendineae located in the left ventricle. A healthy heart pumps blood from the left atrium into the left ventricle, where the mitral valve leaflets ensure that blood flows in one direction and eventually out through the left ventricular outflow tract. Due to the disease of mitral valve leaflets, the mitral valve leaflets cannot be completely closed and cannot completely ensure the one-way flow of blood in the heart, leading to the occurrence of mitral valve regurgitation. The mitral valve leaflets includes a native anterior leaflet and a native posterior leaflet, the two ends of the chordae tendineae of the native posterior leaflet are respectively connected to the native posterior leaflet and the papillary muscle of the native posterior leaflet, and there is a gap between two adjacent chordae tendineae of the native posterior leaflet. Since the part of the mitral valve leaflets that usually suffer from disease is the native posterior leaflet, the diseased native posterior leaflet cannot function normally, resulting in insufficiency of the mitral valve leaflets.

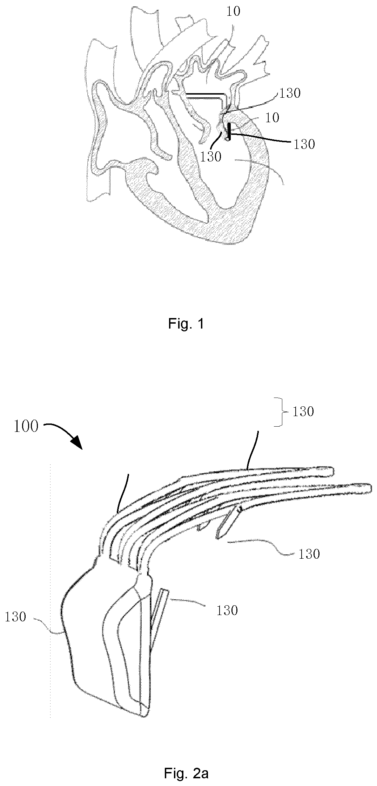

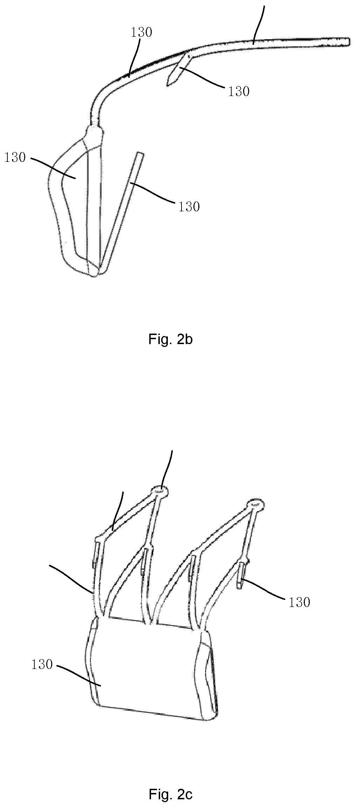

Fig. 1 is a schematic structural diagram of an artificial heart valve located in a heart according to an embodiment of the present invention. Figs. 2a-2c are schematic diagrams of the three-dimensional structure of an artificial heart valve viewed from different perspectives according to an embodiment of the present invention. As shown in Figs. 1 and 2a-2c, this embodiment provides an artificial heart valve 100, which is implanted in the heart via a catheter and eventually anchored between the left atrium 10 and the left ventricle 20. The artificial heart valve 100 replaces part of the mitral valve leaflets to ensure unidirectional blood flow and is used to treat insufficiency of the mitral valve leaflets caused by the diseased native posterior leaflet 21, i.e., to prevent blood from flowing back from the left ventricle 20 into the left atrium 10.

The artificial heart valve 100 includes a main stent 110 and an artificial valve leaflet 120 that are connected in the longitudinal direction. Here, the main stent 110 and the artificial valve leaflet 120 are arranged in sequence from the inflow end to the outflow end of the artificial heart valve 100, that is, the outflow end of the main stent 110 is connected to the inflow end of the artificial valve leaflet 120.

After the artificial heart valve 100 is implanted in the heart, the main stent 110 is fixed on the mitral valve annulus above the native posterior leaflet 21, and the artificial valve leaflet 120 is fixed on the native posterior leaflet 21. Specifically, the artificial valve leaflet 120 is sealed against the inner side of the native posterior leaflet 21 of the mitral valve leaflets (that is, the side of the native posterior leaflet 21 facing the native anterior leaflet 22), and is used to replace the native posterior leaflet 21 to mate with the native anterior leaflet 22 to ensure the unidirectional flow of blood in the left atrium 10 and the left ventricle 20. It should be noted that the "longitudinal" refers to the direction parallel to the blood flow direction after the artificial heart valve 100 is implanted in the heart, and may also refer to the direction parallel to the axis of the mitral valve annulus after the artificial heart valve 100 is implanted in the heart. In this way, the direction perpendicular to the blood flow direction or the axis of the mitral valve annulus is the lateral direction of the artificial heart valve 100.

The main stent 110 is used to achieve anchoring of the artificial heart valve 100 to the mitral valve annulus. The main stent 110 has an arc-shaped structure, that is, the longitudinal cross-section of the main stent 110 is an arc segment, and the transverse cross-section is also an arc segment. The main stent 110 is relatively small in size, so the overall volume of the artificial heart valve 100 is small, and it occupies a relatively small space in the area where the mitral valve annulus is after implantation. The main stent 110 protrudes from its inflow end to the outflow end toward the side away from the inner side of the native posterior leaflet 21 and extends in a parabolic shape, wherein the longitudinal straight line A extends along the above-mentioned longitudinal direction, that is, the longitudinal straight line is parallel to the longitudinal direction. The main stent 110 and the artificial valve leaflet 120 are both arranged on one side of the longitudinal straight line A. Here, the side of the main stent 110 facing the native posterior leaflet 21 is defined as the outer side of the main stent 110, and the side of the main stent 110 away from the native posterior leaflet 21 is defined as the inner side of the main stent 110.

The main stent 110 includes a first section 111 and a second section 112 that are connected in the longitudinal direction, the outflow end of the first section 111 is connected to the inflow end of the second section 112, and the inflow end of the first section 111 is configured to connect to the delivery system before release, so that the artificial heart valve 100 can be stably loaded to the distal end of the delivery catheter 30 of the delivery system before release, and it can also assist the artificial heart valve 100 to anchor the main stent 110 on the mitral valve annulus after release. The outflow end of the second section 112 is connected to the inflow end of the artificial valve leaflet 120 to carry the artificial valve leaflet 120.

After the artificial heart valve 100 is implanted in the heart, the first section 111 as a whole gradually protrudes from its inflow end to the outflow end toward the inner side of the main stent 110 and is connected to the inflow end of the second section 112, so that the first section 111 can be anchored above the mitral valve annulus. At the same time, the first section 111 has a smaller size, which is conducive to providing a compact artificial heart valve 100 and reducing the impact of the artificial heart valve 100 on heart function.

The first section 111 includes at least one first fixing lug 1111 and two first ribs 1112 connected to each of the first fixing lugs 1111. The inflow ends of the two first ribs 1112 are connected together and then connected to one of the first fixing lugs 1111. The outflow ends of the two first ribs 1112 are connected to the second section 112. The first ribs 1112 each extend from the inflow end to the outflow end in a curve (specifically, a parabola), and bulges away from the inner side of the native posterior leaflet (i.e., bulges toward the inner side of the main stent 110), so that the first section 111 can be anchored above the mitral valve annulus. The first fixing lug 1111 is, for example, in a circular ring shape, so that a fixing pull wire located at the distal end of the delivery catheter of the delivery system can be passed through the first fixing lug 1111. The number of the first fixing lugs 1111 is, for example, at least one, such as one, two, three, four, etc. Preferably, the number of the first fixing lugs 1111 is two.

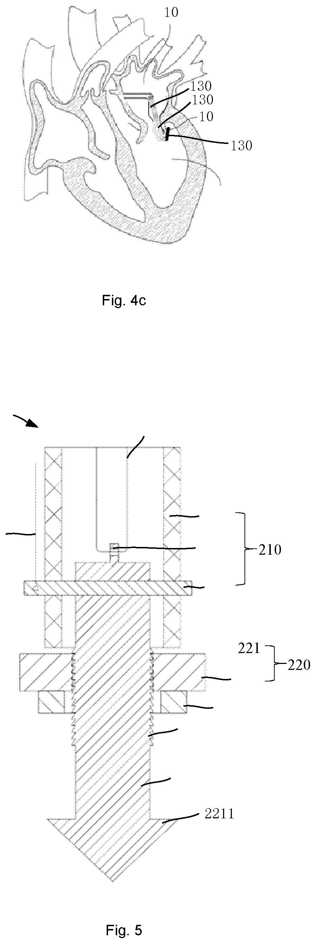

Optionally, Fig. 5 is a schematic diagram of a fixing stent according to an embodiment of the present invention. As shown in Fig. 5, the artificial heart valve 100 also includes at least one fixing stent 200, the outflow end of each of fixing stent 200 is inserted into one of the first fixing lugs 1111, and the inflow end is connected to the delivery system, so that the first fixing lug 1111 is connected to a delivery system through the fixing stent 200. After the artificial heart valve 100 is implanted in the heart, the outflow end of the fixing stent 200 is anchored on the mitral valve annulus above the native posterior leaflet 21, and the inflow end of the first section 111 is fitted and fixed on the mitral valve annulus.

The fixing stent 200 includes a connecting section 210 and an anchoring section 220 that are arranged in the longitudinal direction. The connecting section 210 is detachably connected to the inflow end of the anchoring section 220, and the connecting section 210 is connected to the delivery system.

The anchoring section 220 includes a peg 221 and a pressing sheet 222. The pressing sheet 222 and the first fixing lugs 1111 are sleeved over the peg 221. The pressing sheet 222 is arranged close to the inflow end of the peg 221, and the first fixing lugs 1111 are arranged close to the outflow end of the peg 221. After the artificial heart valve 100 is implanted in the heart, the outflow end of the peg 221 pierces the native tissue of the mitral valve annulus. At this time, the first fixing lug 1111 is longitudinally limited on the pin between the native tissue and the pressing sheet 222, so that the inflow end of the main stent 110 is arranged in contact with the native tissue.

The peg 221 is cylindrical, and the outflow end of the peg 221 has a sharp end 2211, so that the peg 221 is roughly arrow-shaped. Particularly, the peg 221 includes a cylinder and a cone that are connected in the longitudinal direction, wherein the bottom surface of the cone is connected to a bottom surface of the cylinder, and the diameter of the cylinder is smaller than the diameter of the bottom surface of the cone, and the diameter of the bottom surface of the cone is larger than the inner diameter of the first fixing lug 1111, so that the first fixing lug 1111 will not detach from a side where the outflow end of the peg 221 is.

The outer circumferential surface of the peg 221 has a serrated section 2212, and the serrated section 2212 is located in the middle area of the peg 221 along the longitudinal direction. The serrated section 2212 has a serrated structure arranged along the longitudinal direction, and the pressing sheet 222 is annular (for example, a circular ring), and the side wall of the inner ring of the pressing sheet 222 has a serrated structure matching the serrated structure of the serrated section 2212, so that after the pressing sheet 222 is sleeved on the peg 221 from the inflow end of the peg 221, it can only move unidirectionally from the inflow end to the outflow end of the serrated section 2212, thereby achieving self-locking.

The connecting section 210 includes a second fixing lug 211, a sleeve 212 and a pin 213. The second fixing lug 211 is fixed on the end face of the inflow end of the peg 221 and is configured to connect the fixing pull wire 31 of the delivery system. The pin 213 fixes the sleeve 212 on the inflow end of the peg 221. The sleeve 212 is sleeved over the inflow end of the peg 221, and the side wall of the inflow end of the peg 221 has a first through hole (not shown in the figures) that passes through the peg 221 laterally, and the side wall of the outflow end of the sleeve 212 has two second through holes (not shown in the figures) that pass through the sleeve 212 laterally. The pin 213 is, for example, cylindrical, and the first through hole and the second through hole can be circular, and the diameter of the pin 213 is smaller than the smallest diameter of the first through hole and the second through hole. Preferably, the first through hole and the second through hole have the same shape and size, and the diameter of the pin 213 is slightly smaller than the diameter of the first through hole. When the sleeve 212 is sleeved on the inflow end of the peg 221, the center of the first through hole and the center of the two second through holes are on a same straight line, and the pin 213 is inserted through the first through hole and the second through holes, so that the sleeve 212 is fixed by the pin 213 and the peg 221, and at least one end of the pin 213 extends out of the outer circumferential surface of the sleeve 212, and one end of the pin 213 is connected to the unlocking pull wire 32 of the delivery system, so that the unlocking pull wire 32 can withdraw the pin 213 from the second through holes of the sleeve 212 and the first through hole of the peg 221 to destroy the fixedly connection between the sleeve 212 and the peg 221. At this time, the sleeve 212 is withdrawn along with the delivery catheter of the delivery system, and the pressing sheet 222 and the peg 221 as a whole anchor the main stent 110 on the mitral valve annulus above the native posterior leaflet 21.

Please continue to refer to Figs. 2a-2c, the second section 112 includes a plurality of second ribs 1121. After all the second ribs 1121 are connected end to end in sequence, the inflow end of each second rib is connected to the outflow end of one of the first ribs 1112, so that the outflow ends of some of the first ribs 1112 each can be connected to the inflow ends of two second ribs 1121 at the same time, and the outflow ends of the remaining first ribs 1112 each can be connected to the inflow end of one second rib 1121. The two first ribs 1112 connected to each first fixing lug 1111 and the two corresponding second ribs 1121 form a closed mesh cell, which for example, is prismatic. The outflow ends of the second ribs 1121 are connected to the inflow end of the artificial valve leaflet 120.

In this embodiment, the first section 111 includes two first fixing lugs and four first ribs 1112, and the second section 112 includes six second ribs 1121. After the inflow ends of every two first ribs 1112 are connected, the outflow ends of all the first ribs 1112 are arranged in sequence in the horizontal direction. After the six second ribs 1121 are connected end to end in sequence, the inflow ends of the four second ribs 1121 located in the middle in the horizontal direction are connected in pairs in sequence and then connected to the outflow ends of the two first ribs 1112 located in the middle in the horizontal direction respectively. The two second ribs 1121 located at both ends in the horizontal direction are not connected to the inflow ends of the adjacent second ribs 1121, and are separately connected to the outflow ends of the two first ribs 1112 located on both sides in the horizontal direction. After the outflow ends of the six second ribs 1121 are connected in pairs, they are respectively connected to the inflow ends of the artificial valve leaflet 120, so that the two first ribs 1112 connected to each first fixing lug 1111 are respectively connected with the two second ribs 1121 opposite to the two first ribs 1112 to form a closed prismatic mesh cell.

The main stent 110 further includes a plurality of barbs 140, all of which are disposed on the outer side of the main stent 110, so that all of the barbs 140 are disposed toward the native posterior leaflet 21 after the artificial heart valve 100 is implanted in the heart. Each of the barbs 140 is arranged at the outflow end of the first section 111 and the inflow end of the second section 112, that is, each of the barbs 140 is arranged at the outflow end of one of the first ribs 1112 and the inflow end of one of the second ribs 1121, and is configured to realize the anchoring of the artificial heart valve 100 to the mitral valve annulus, so that, after the artificial heart valve 100 is implanted in the human body, the second section 112 can be fixed on the mitral valve annulus with the help of the barbs 140, thereby preventing the artificial heart valve 100 from falling from the left atrium into the left ventricle.

In this embodiment, the inflow ends of the barbs 140 are connected to the outflow ends of the first ribs 1112 and the inflow ends of the second ribs 1121. The outflow ends of the barbs 140 are free ends, and an acute angle is formed between the barbs 140 and the second section 112. In other embodiments, the outflow ends of the barbs 140 are connected to the outflow ends of the first ribs 1112 and the inflow ends of the second ribs 1121. The inflow ends of the barbs 140 are free ends, and an acute angle is formed between the barbs 140 and the first section 111.

The main stent 110 is formed by braiding or cutting and made of nickel-titanium alloy or other biocompatible materials with shape memory properties.

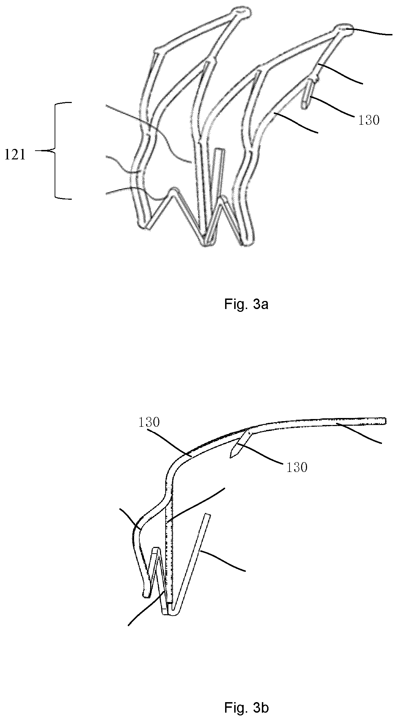

Figs. 3a-3b are schematic diagrams of the three-dimensional structure of a stent body and a leaflet stent according to an embodiment of the present invention. As shown in Figs. 3a-3b, and also refer to Figs. 2a-2c, the artificial valve leaflet 120 includes a valve leaflet stent 121, a covering and a clamping component 130, wherein the inflow end of the valve leaflet stent 121 is connected to the outflow end of the second section 112, and the covering covers the outer surface of the valve leaflet stent 121, so that the artificial valve leaflet 120 is configured to seal and abut against the inner side of the native posterior leaflet 21 of the mitral valve leaflets, and mate with the native anterior leaflet 22 of the mitral valve leaflets. The clamping component 130 is fixed to the side of the valve leaflet stent 121 away from the native anterior leaflet, and the covering exposes the clamping component 130. The clamping component 130 is configured to clamp the native posterior leaflet 21 to provide an anchoring effect to prevent the leaflet from being impacted into the left atrium by blood flow when the leaflet is closed. Specifically, the clamping component 130 is arranged on the same side as the barbs 140 and is located at the outflow end of the valve leaflet stent 121, and is configured to clamp and fix the native posterior leaflet 21 between the clamping component 130 and the valve leaflet stent 121.

After the artificial heart valve 100 is implanted in the heart, the artificial valve leaflet 120 is relatively stationary and is closed or merged by sealingly abutting with the native posterior leaflet 21 to replace the native posterior leaflet 21 so as to mate with the native anterior leaflet 22 to ensure the unidirectional flow of blood in the left atrium 10 and the left ventricle 20. At the same time, the clamping component 130 passes through the tendineae under the native posterior leaflet 21 and clamps the native posterior leaflet 21 between the valve leaflet stent 121 and the clamping component 130, so as to anchor the valve leaflet stent 121 covered with the covering (i.e., the artificial valve leaflet 120) on the inner side of the native posterior leaflet 21. The main stent 110 is anchored on the mitral valve annulus through the barbs 140. The anchoring of the barbs 140 and the clamping component 130 prevents the artificial heart valve 100 from shifting under the impact of blood flow, so that the artificial heart valve of the present invention has a compact structure and a small size. After implantation, it only occupies the area where the posterior leaflet is, has little effect on the function of the heart, and occupies a small area of the valve orifice, so the impact of blood flow is small, which can increase the lifespan of the artificial heart valve.

Furthermore, the surface of the artificial valve leaflet 120 facing the native anterior leaflet 22 (i.e., the mating surface of the artificial valve leaflet with the native anterior leaflet 22) matches in shape with the surface of the native posterior leaflet 21 facing the native anterior leaflet 22 (i.e., the mating surface of the native posterior leaflet 21 with the native anterior leaflet 22), so that the surface of the artificial valve leaflet 120 facing the native anterior leaflet 22 can replace the native posterior leaflet 21 and mate with the native anterior leaflet. Therefore, the mating surface of the artificial valve leaflet 120 can be approximate or similar in shape to the mating surface of the native posterior leaflet 21.

Specifically, the leaflet stent 121 is formed by a single-layer stent, and the valve leaflet stent 121 is constructed into a three-dimensional structure by combining convex and concave shapes in the transverse direction. The three-dimensional structure has a cavity along the transverse direction, so that the covering that covers the valve leaflet stent 121 forms a pocket-like structure in the cavity, which is beneficial for making the surface of the artificial valve leaflet 120 facing the native anterior leaflet 22 to be similar in shape to the surface of the native posterior leaflet 21 facing the native anterior leaflet 22. The valve leaflet stent 121 includes at least two sub-stents connected in sequence along the transverse direction, and the adjacent ones of the sub-stents are symmetrically arranged with respect to the connection points. Each of the sub-stents includes a third rib 1211, a fourth rib 1212 and a fifth rib 1213, the inflow end of the third rib 1211 and the inflow end of the fourth rib 1212 are spaced apart and are both connected to the outflow end of the second section 112, the outflow end of the third rib 1211 and the outflow end of the fourth rib 1212 are spaced apart and are respectively connected to the two ends of the fifth rib 1213. The fifth rib 1213 may have a V-shape formed by connecting two ribs in sequence, or a U-shape, a W-shape, a Z-shape etc., each of which is formed by connecting at least two ribs in sequence, or it can be a single rib arranged horizontally.

Taking the V-shape as an example, the fifth rib 1213 has a vertex and two free ends, the vertex is at the ends of the two ribs that are connected to each other, and the two free ends are the ends of the two ribs that are not connected to each other, one of the free ends of the fifth rib 1213 is connected to the outflow end of the third rib 1211, and the other one of the free ends of the fifth rib 1213 is connected to the outflow end of the fourth rib 1212, and the vertex is located between the third rib 1211 and the fourth rib 1212 in the horizontal direction.

In two adjacent sub-stents, the third rib 1211 of one of the sub-stents is connected to the third rib 1211 of the other one of the sub-stents along the transverse direction, and the two sub-stents can be symmetrically arranged with respect to the third rib 1211 that serves as the axis of symmetry; the fourth rib 1212 of one of the sub-stents is connected to the fourth rib 1212 of the other one of the sub-stents along the transverse direction, and the two sub-stents can also be symmetrically arranged with respect to the fourth rib 1212 that serves as the axis of symmetry. After the inflow end of the third rib 1211 and the inflow end of the fourth rib 1212 are respectively connected to the outflow end of the second section 112, at least one of the third rib 1211 and the fourth rib 1212 bulges away from the other in the transverse direction.

Furthermore, the third rib 1211 is straight (i.e., it extends straightly from the inflow end to outflow end thereof) or curved (i.e., it extends curvedly from the inflow end to outflow end thereof), and the curved rib bulges away from the fourth rib. The fourth rib 1212 is curved, and the fourth rib bulges away from the third rib. Specifically, the fourth rib 1212 first extends away from the third rib 1211 in the transverse direction and away from the outflow end of the second section 112 in the longitudinal direction, and then extends toward the third rib 1211 in the transverse direction and away from the outflow end of the second section in the longitudinal direction.

In this embodiment, the valve leaflet stent 121 includes two sub-stents connected in sequence along the transverse direction. After the third ribs of the two sub-stents are connected, the fourth rib and the fifth rib of each sub-stent are symmetrically arranged on both sides of the third rib with respect to the third rib that serves as the symmetry axis. The two sub-stents are respectively connected to the outflow ends of the two second ribs located in the middle along the transverse direction, and the inflow ends of the two third ribs are connected, the inflow end of each fourth rib is connected to the outflow ends of the two second ribs connected to each other, the outflow end of the third rib is connected to a free end of the fifth rib, the outflow end of the fourth rib is connected to the other free end of the fifth rib, and the third rib is arranged close to the surface of the inner side of the native posterior leaflet, the fourth rib is arranged close to the surface of the inner side of the native anterior leaflet, and the vertex of the fifth rib is located between the inflow end and the outflow end of the fourth rib along the longitudinal direction, so that the third rib, the fourth rib and the fifth rib together form a three-dimensional structure in the transverse direction, and the transverse gap between the two groups of third ribs and fourth ribs forms a cavity.

The third rib 1211, the fourth rib 1212 and the fifth rib 1213 of each sub-stent may be connected by welding or by integral molding, and all the sub-stents of the valve leaflet stent 121 may be connected by welding or by integral molding.

The valve leaflet stent 121 may be made of metal material or a polymer material with relatively high rigidity. The covering may be made of heart valves or pericardial tissue of animals (such as pigs, sheep, horses, and cows), or synthetic materials, such as expanded polytetrafluoroethylene or polyester; it may also be made of thermoplastic polycarbonate polyurethane, polyether polyurethane, segmented polyether polyurethane, silicone polyether polyurethane, silicone-polycarbonate polyurethane or ultra-high molecular weight polyethylene; it can also be made of polyolefins, elastomers, polyethylene glycol, polyether sulfone, polysulfone, polyvinyl pyrrolidone, polyvinyl chloride, fluoropolymers, silicone polyesters, siloxane polymers, siloxane oligomers, polylactones, or block copolymers of at least two of the above materials.

Optionally, the surface of the covering is treated with or reacted with an anticoagulant, and the anticoagulant includes but is not limited to heparinized polymers.

The covering covers the valve leaflet stent 121 so that the covering has a pocket-like shape, and a filler is filled in the cavity. After the filler is filled, the opening of the covering is closed. The filler may be biocompatible foam or the like. Optionally, the biocompatible foam includes medical foam silicone rubber, medical polyurethane foam or the like.

The clamping component 130 is fixed to a side of the outflow end of the valve leaflet stent 121, and the clamping component 130 and the barbs 140 are arranged on the same side. Specifically, the clamping component 130 is, for example, formed by bending a metal rod, and the clamping component 130 has two ends, one end of which is connected to the outflow end of the valve leaflet stent 121, and the other end may extend away from the valve leaflet stent 121 in the transverse direction and away from the inflow end of the valve leaflet stent 121 in the longitudinal direction, and then extend away from the valve leaflet stent 121 in the transverse direction and toward the inflow end of the valve leaflet stent 121 in the longitudinal direction, to ensure the area range of the native posterior leaflet 21 for being clamped. In this embodiment, one end of the clamping component 130 is connected to the outflow ends of two adjacent third ribs and is located outside the valve leaflet stent 121.

The clamping component 130 may be connected to the valve leaflet stent 121 by welding, and can also be integrally formed with the valve leaflet stent 121. The clamping component 130 may be made of nickel-titanium alloy or other biocompatible materials with shape memory properties, or can be made of elastically or plastically deformable materials, such as balloon-expandable materials, so that the clamping component 130 has shape memory properties to achieve the clamping and fixation action on the native posterior leaflet 21.

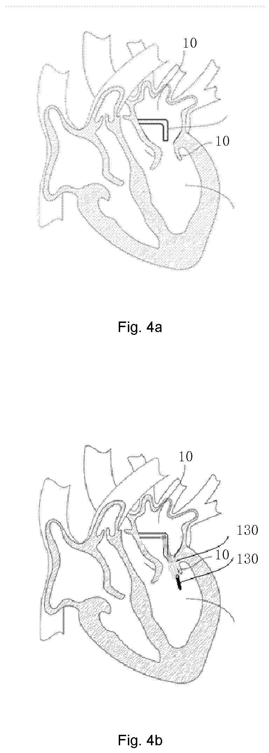

Figs. 4a-4c are schematic diagrams of an artificial heart valve during implantation according to an embodiment of the present invention. Reference is made to Figs. 4a-4c, and Fig. 1. During the implantation of the artificial heart valve 100, as shown in Fig. 4a, the artificial heart valve 100 is loaded on the distal end of the delivery catheter 30 of the delivery system, and the distal end of the delivery catheter 30 is inserted into the second fixing lug 211. The delivery catheter 30 enters the right atrium through the inferior vena cava, and passes through the atrial septum to reach the vicinity of the mitral valve annulus of the left atrium 10, so that the artificial heart valve 100 is implanted with less trauma. At this time, the first fixing lug 1111 is sleeved on the peg 221 of the fixing stent 200, and is located between the sharp end 2211 and the pressing sheet 222, the pin 213 fixes the sleeve 212 on the peg 221, and the fixing pull wire 31 of the delivery system passes through the second fixed lug 211, and the unlocking pull wire does not provide a pulling force to the pin 213; then, as shown in Fig. 4b, the delivery catheter 30 begins to release the artificial heart valve 100, and the outflow end of the artificial valve leaflet 120 is released first, and the clamping component 130 located at the outflow end of the valve leaflet stent 121 is released to cross the native posterior leaflet 21. At this time, the clamping component 130 is not in effect and is parallel to the native posterior leaflet 21. As shown in Fig. 4c, with the release of the artificial valve leaflet, the clamping component 130 begins to bend and gradually passes through the tendons and clamps the native posterior leaflet 21 between the clamping component 130 and the artificial valve leaflet 120. At this time, the artificial valve leaflet 120 has occupied the space for the native posterior leaflet 21 to mate with the native anterior leaflet 22, and the native anterior leaflet 22 begins to mate with the relatively static artificial valve leaflet 120; as shown in Fig. 1, when the clamping component 130 stably clamps the native posterior leaflet 21 and the artificial valve leaflet 120 reaches a position for fixing, the main stent 110 is released on the mitral valve annulus, and the barbs on the main stent 110 are inserted into the mitral valve annulus to release and fix the anchoring section 220. Specifically, since the position of the main stent 110 has been determined, the fixing stent 200 begins to move. The sleeve moves toward the mitral valve annulus, and the sharp end 2211 of the peg 221 pierces the native tissue on the mitral valve annulus and is anchored on the mitral valve annulus; then the unlocking pull wire provides a pulling force to the pin 213, so as to withdraw the pin 213 by utilizing the unlocking pull wire, and the sleeve 212 is pushed and continuously moved toward the mitral valve annulus, so that the pressing sheet 222 and the first fixing lug 1111 move longitudinally from the inflow end to the outflow end of the serrated section 2212, that is, the pressing sheet 222 and the first fixing lug 1111 move toward the sharp end 2211, so that the inflow end of the main stent 110 is attached to the native tissue surface on the mitral valve annulus. At this time, due to the self-locking effect of the serrated section 2212, the peg 221, the pressing sheet 222 and the main stent 110 are fixed together. At this time, under the joint action of the clamping component 130, the barbs 140 and the anchoring section 220, the main stent 110 is relatively fixed to the mitral valve annulus, and at the same time, the second section 112 is fixed to the mitral valve annulus of the heart with the help of the barbs 140; then the fixing pull wire located at the distal end of the delivery catheter 30 is withdrawn, and the sleeve is taken out at the same time, the anchoring section is released, and the delivery system is withdrawn to complete the release.

In summary, the present invention provides an artificial heart valve, which clamps the native posterior leaflet by barbs and clamping component, so that the artificial heart valve is more reliably anchored between the left atrium and the left ventricle. The artificial valve leaflet replaces the native posterior leaflet and mates with the native anterior leaflet, so the healthy native anterior leaflet can be effectively utilized, thereby improving the mating performance and lifespan of the artificial valve leaflet. The artificial heart valve of the present invention has a compact structure and a small size. After implantation, it only occupies the area where the posterior leaflet is, has little effect on the function of the heart, and occupies a small area of the valve orifice so that the impact force of blood flow is small, which can increase the lifespan of the artificial heart valve. Additionally, artificial heart valves can be implanted via a transseptal approach, making implantation less invasive.

In addition, it should be noted that, unless otherwise specified or indicated, the terms "first" and "second" in the specification are only used to distinguish the various components, elements, steps, etc. in the specification, and are not used to indicate the logical relationship or sequential relationship between the various components, elements, steps, etc.

It is to be understood that, although the present invention has been disclosed above with reference to preferred embodiments, the above embodiments are not intended to limit the present invention. For any technician familiar with the field, without departing from the scope of the technical solution of the present invention, the technical content disclosed above can be used to make many possible changes and modifications to the technical solution of the present invention, or to modify it into an equivalent embodiment with equivalent changes. Therefore, any simple modifications, equivalent changes and modifications made to the above embodiments based on the technical essence of the present invention without departing from the content of the technical solution of the present invention shall still fall within the scope of protection of the technical solution of the present invention.

## Claims

1. An artificial heart valve, comprising a main stent and an artificial valve leaflet, wherein an outflow end of the main stent is connected to an inflow end of the artificial valve leaflet, and when the artificial heart valve is implanted in the heart, the main stent is fixed on a mitral valve annulus above a native posterior leaflet of mitral valve leaflets, and the artificial valve leaflet is sealed against an inner side of the native posterior leaflet and mates with a native anterior leaflet of the mitral valve leaflets to ensure a unidirectional flow of blood in a left atrium and a left ventricle;
wherein, the artificial valve leaflet comprises a valve leaflet stent and a covering, and wherein the valve leaflet stent is a single-layer three-dimensional stent, and the covering covers the valve leaflet stent.

2. The artificial heart valve according to claim 1, wherein the main stent has an arc-shaped structure; when the artificial heart valve is implanted in the heart, a surface of the artificial valve leaflet facing the native anterior leaflet matches in shape with a surface of the native posterior leaflet facing the native anterior leaflet.

3. The artificial heart valve according to claim 2, wherein the main stent comprises a first section and a second section that are connected along a longitudinal direction, an inflow end of the first section is configured to be connected to a delivery system before the artificial heart valve is released, an outflow end of the first section is connected to an inflow end of the second section, and an outflow end of the second section is connected to an inflow end of the valve leaflet stent.

4. The artificial heart valve according to claim 3, wherein the first section comprises at least one first fixing lug and two first ribs connected to each of the at least one first fixing lug, inflow ends of the two first ribs are connected together to one of the at least one first fixing lug, outflow ends of the two first ribs are respectively connected to the second section, the first fixing lug is configured to connect to the delivery system, and the outflow ends of the first ribs are connected to the inflow end of the second section.

5. The artificial heart valve according to claim 4, wherein each of the first ribs extends curvedly from the inflow end to the outflow end of the first rib and bulges away from the inner side of the native posterior leaflet.

6. The artificial heart valve according to claim 4, wherein the second section comprises a plurality of second ribs, and wherein after all the second ribs are connected end to end in sequence, inflow ends of the second ribs are connected to the outflow ends of the first ribs, and outflow ends of the second ribs are connected to the inflow end of the valve leaflet stent, so that the two first ribs connected to a same one of the at least one first fixing lug and corresponding two second ribs together form a closed mesh cell.

7. The artificial heart valve according to claim 4, further comprising at least one fixing stent, wherein each of the at least one fixing stent has an outflow end inserted into one of the at least one first fixing lug, and an inflow end connected to the delivery system;
after the artificial heart valve is implanted in the heart, the outflow end of the fixing stent is anchored on the mitral valve annulus above the native posterior leaflet, and the inflow end of the first section is fitted and fixed on the mitral valve annulus.

8. The artificial heart valve according to claim 7, wherein the fixing stent comprises a connecting section and an anchoring section that are arranged in a longitudinal direction, wherein the connecting section is detachably connected to an inflow end of the anchoring section, and the connecting section is connected to the delivery system.

9. The artificial heart valve according to claim 8, wherein the anchoring section comprises a peg and a pressing sheet, wherein the pressing sheet and the at least one first fixing lug are sleeved over the peg, the pressing sheet is arranged close to an inflow end of the peg, and the at least one first fixing lug is arranged close to an outflow end of the peg;
after the artificial heart valve is implanted in the heart, the outflow end of the peg pierces a native tissue of the mitral valve annulus, the at least one first fixing lug is longitudinally limited on the peg between the native tissue and the pressing sheet, and an inflow end of the main stent is arranged in contact with the native tissue.

10. The artificial heart valve according to claim 9, wherein the outflow end of the peg has a sharp end, the peg has a largest cross-sectional diameter at an inflow end of the sharp end, and a smallest cross-sectional diameter at an outflow end of the sharp end.

11. The artificial heart valve according to claim 9, wherein a serrated section is provided on a middle area of an outer circumferential surface of the peg, and the serrated section has a serrated structure; the pressing sheet is annular, and a side wall of an inner ring has a serrated structure matching the serrated structure of the serrated section, so that the pressing sheet can only move unidirectionally from an inflow end to an outflow end of the serrated section.

12. The artificial heart valve according to claim 8, wherein the connecting section comprises a second fixing lug, a sleeve and a pin, wherein the second fixing lug is fixed to an end face of an inflow end of the peg and is configured to connect to a fixing pull wire of the delivery system, the sleeve is sleeved over an inflow end of the peg and is connected to the peg through the pin, and an end of the pin is connected to an unlocking pull wire of the delivery system.

13. The artificial heart valve according to claim 12, wherein a first through hole extending through the peg laterally is defined on a side wall of the inflow end of the peg, two second through holes extending through the sleeve laterally is defined on a side wall of an outflow end of the sleeve, the pin is inserted into the first through hole and the second through hole, and can be withdrawn from the first through hole and the second through hole through the unlocking pull wire.

14. The artificial heart valve according to claim 1, wherein the main stent comprises a plurality of barbs, and the artificial valve leaflet further comprises a clamping component, wherein all of the barbs and the clamping component are arranged on a same side, and the barbs are arranged at an outflow end of a first section of the main stent and an inflow end of the second section of the main stent, the clamping component is arranged at an outflow end of the valve leaflet stent, and the covering exposes the clamping component,
when the artificial heart valve is implanted in the heart, the main stent is anchored on the mitral valve annulus with an assistance of the barbs, and the clamping component clamps and fixes the native posterior leaflet between the clamping component and the valve leaflet stent.

15. The artificial heart valve according to claim 1, wherein the valve leaflet stent comprises at least two sub-stents connected in sequence along a transverse direction, and two adjacent ones of the at least two sub-stents are symmetrically arranged with respect to a connection point.

16. The artificial heart valve according to claim 15, wherein each of the at least two sub-stent comprises a third rib, a fourth rib and a fifth rib, wherein an inflow end of the third rib and an inflow end of the fourth rib are arranged at intervals and are both connected to an outflow end of a second section of the main stent, the fifth rib is located between the third rib and the fourth rib in the transverse direction, and an outflow end of the third rib and an outflow end of the fourth rib are respectively connected to two ends of the fifth rib.

17. The artificial heart valve according to claim 16, wherein the third rib bulges away from the fourth rib, and/or the fourth rib bulges away from the third rib.

18. The artificial heart valve according to claim 17, wherein the third rib is straight, and the fourth rib is curved, wherein the fourth rib extends away from the third rib in the transverse direction and away from the outflow end of the second section in a longitudinal direction, and then extends toward the third rib in the transverse direction and away from the outflow end of the second section in the longitudinal direction.

19. The artificial heart valve according to claim 1, wherein the covering is filled with a filler.

20. The artificial heart valve according to claim 1, wherein the clamping component is formed by bending a metal rod, and the clamping component has two ends, one end of which is connected to an outflow end of the valve leaflet stent, and the other end extends away from the valve leaflet stent in a transverse direction and away from an inflow end of the valve leaflet stent in a longitudinal direction, and then extends away from the valve leaflet stent in the transverse direction and toward the inflow end of the valve leaflet stent in the longitudinal direction.
